# EUROPEAN PATENT SPECIFICATION

(11) **EP 2 265 191 B1**
(45) Date of publication and mention of the grant of the patent: **29.02.2012**
(21) Application number: 08723936.4
(22) Date of filing: 03.04.2008
(51) Int. Cl.: A61B 17/11, A61B 17/122

(54) **SECURING DEVICE AND ASSEMBLY COMPRISING SUCH A SECURING DEVICE**
FIXIERUNGSVORRICHTUNG UND ANORDNUNG MIT EINER SOLCHEN FIXIERUNGSVORRICHTUNG
DISPOSITIF DE FIXATION ET ASSEMBLAGE COMPRENANT CE TYPE DE DISPOSITIF DE FIXATION

(43) Date of publication of application: 29.12.2010
(73) Proprietor: AMJ B.V., 3584 CM Utrecht (NL)
(72) Inventor: TULLEKEN, Cornelis Antonius Franciscus, NL-3511 XP Utrecht (NL); MANSVELT BECK, Hendricus Jacobus, NL-3941 EA Doorn (NL); VAN DOORMAAL, Theodorus Petrus Cornelis, NL-3732 DK De Bilt (NL)
(74) Representative: Groeneveld, Yme Geert
(86) International application number: PCT/NL2008/050185
(87) International publication number: WO 2009/123434

(56) References cited:
- US-A1- 2006 025 790

## Description

The present invention relates to a securing device for an anastomosis connection, comprising a ring;
in which the ring has an axial axis which is defined as an axis at right angles to the annular plane surrounded by the ring and through the center of the ring;
in which the ring has a first transverse axis and a second transverse axis, which are at right angles to one another and are both also at right angles to the axial axis, and which together define a transverse plane;
in which the first transverse axis divides the ring into a first half and a second half;
in which the transverse plane has a first side along which the first half of the ring extends and a second side along which the second half of the ring extends.

Although it is not a securing device, a ring of this type is know for use with an anastomosis connection. A ring of this type is disclosed in EP 750,476. Said publication describes what is referred to as the ELANA technique (Elana = Excimer Laser Assisted Nonocclusive Anastomosis). The present invention is particularly intended for use with the ELANA technique, but can also be used for other medical procedures.

The ELANA technique is used to produce an anastomosis without temporarily closing off the receiving vessel, also referred to as target vessel. With the ELANA technique, the through-flow of the target vessel is not interrupted while the anastomosis is performed. Neurosurgeons, as well as other surgeons, use this technique to perform bypass operations. However, this ELANA technique can also be applied in other areas of surgery, such as cardiac surgery and general vascular surgery. The present invention can also be used in the case of these medical procedures.

The ELANA technique involves placing an implantable ring around the distal end of a graft vessel, folding the section of the wall of the graft vessel, which is distal from the ring, back over approximately 90° or approximately 180° around the ring in order to form a distal mouth of the graft vessel reinforced by the ring. Said reinforced distal mouth is then attached to the wall of the target vessel, usually by suturing the graft vessel to the target vessel by means of approximately 8 stitches. Depending on the ability of the surgeon, the location of the target vessel and the condition of the patient, said attachment generally takes between 15 minutes and one hour or more. During this attachment procedure, there is still no through-flow connection between the graft vessel and the target vessel. After the attachment procedure, a laser catheter is inserted into the graft vessel in order to create an opening between the graft vessel and the target vessel by burning away the wall section of the target vessel which is situated in front of the distal mouth of the graft vessel. Blood flows through the target vessel during the entire operation, as a result of which temporary closure of the target vessel is prevented.

US 2006/0025790 discloses a securing device comprising a wire element comprising a partial ring forming a cantilevered pin at each and of said partial ring.

It is an object of the present invention to provide a securing device by means of which the procedure of attaching the graft vessel to the target vessel can be carried out in a relatively quick and easy manner, and by means of which a reliable connection can be achieved.

According to the invention, this object is achieved by providing a securing device for an anastomosis connection, comprising a ring provided with two cantilever pins;
in which the ring has an axial axis which is defined as an axis at right angles to the annular plane surrounded by the ring and through the center of the ring;
in which the ring has a first transverse axis and a second transverse axis, which are at right angles to one another and are both at right angles to the axial axis, and which together define a transverse plane;
in which the first transverse axis divides the ring into a first half and a second half;
in which the transverse plane has a first side along which the first half of the ring extends and a second side along which the second half of the ring extends;
in which each cantilever pin, at in each case one attachment site, is connected to the ring by a fixed end in order, in a cantilevered manner, to extend from the ring to the other, free end of the pin, which free end is pointed;
in which, viewed from the ring, the pins extend in the same direction in the transverse plane;
in which, viewed in the transverse plane, the free ends of the pins are situated on the second side of the transverse plane;
in which the attachment sites are situated on the first half of the ring.
The first half of the ring also includes the boundary thereof, that is to say the first transverse axis, so that one or both attachment sites can also be on the first transverse axis.
The pointed ends of the pins point in the same direction so that they can readily pierce the wall of the target vessel together to end up in the target vessel and then to run in the length direction of the target vessel along the inside of the wall of the target vessel. As the fixed ends of the pins, that is to say the attachment sites, are situated on the first half of the ring, as the pins extend from there underneath the second half of the ring, past said second half, and as, in the position where they are pierced into the target vessel, a section of the wall of the target vessel is situated between the second half of the ring and the pins, the wall of the target vessel provides resistance against tilting of the securing device - with respect to the target vessel - about a tilting axis which runs parallel to the first transverse axis. In addition, the securing device provides a resistance against tilting - with respect to the target vessel - about some other tilting axis. Thus, a relatively stable connection can be achieved.

The attachment by means of the securing device according to the invention can be improved further, if the sections of the pins extending along the second side of the transverse plane have a tapering zone in which said pins, viewed in the direction of the free ends thereof, taper towards one another. A first advantage thereof is that, as a result of the tapering, the pierced holes in the wall of the target vessel, which were caused by piercing the pointed ends through the wall, will be pulled apart when the pins are pushed further inwards through the pierced holes. In this connection, it has been found that the pins also keep the section of the target vessel between the latter taut after the securing device has been placed. Thus, the wall of the target vessel, in the region of the second half of the ring, will be pulled taut in a direction parallel to the first transverse axis. This facilitates the removal of the section of the wall of the target vessel which is situated in front of the distal mouth of the graft vessel. According to the ELANA technique, a laser can be used for the removal of said section of the wall of the target vessel, but another instrument, such as a knife, can also be used. A second advantage is that, as a result of said tension, the securing device will not readily become dislodged (i.e. slide out again in a direction opposite to the insertion direction). A third advantage is that as a result of said tension, the above-described tilting of the securing device with respect to the target vessel is prevented further. A fourth advantage is that, as a result of the pointed ends, due to the tapering, being closer to one another, said pointed ends can be more readily pierced into a bulging target vessel - through which the blood or another medium is still flowing. After all, the circular arch of the wall of the target vessel - which arch extends between the piercing locations - will be shorter. The target vessel then does not have to be flattened to such a large degree during piercing.

It is furthermore advantageous in this connection if, in said tapering zone, the perpendicular projection of said pins on said transverse plane at least partially coincides with the perpendicular projection of the ring on said transverse plane and does not fall within the perpendicular projection of the ring on said transverse plane. The result thereof is, on the one hand, that the pins, viewed in the axial direction, partially exactly overlap the ring and do not obstruct the passageway of the graft vessel to the target vessel, which passageway is to be formed later, and, on the other hand, that the pins do not flatten the target vessel to an excessive degree at the location of the anastomosis which is to be formed. It should be noted here that if the ring is flat and extends in the transverse plane, the projection of the ring on the transverse plane then coincides with the ring itself.

According to a further embodiment of the invention, the attachment site of both pins is situated at a distance from the first transverse axis. This results in the wall of the target vessel also being pulled taut in a section of the first half of the ring in a direction parallel to the first transverse axis. In order, in this case, to pull the wall of the target vessel in a relatively large section of the first half of the ring, it is advantageous according to the invention if said distance to the first transverse axis is at least 10% of the radius of the ring, in particular at least 25% of the radius of the ring, more particularly at least 50% of the radius of the ring, even more particularly at least 70% of the radius of the ring.

In this case, it is furthermore advantageous if, in the first side of the transverse plane, the perpendicular projection of the pins on said transverse plane coincides with the perpendicular projection of the ring on said transverse plane. The result thereof is that, on the one hand, the pins, viewed in the axial direction, partially exactly overlap the ring and do not obstruct the passageway of the graft vessel to the target vessel, which passageway is to be formed later, and, on the other hand, that the pins do not flatten the target vessel to an excessive degree at the location of the anastomosis which is to be formed. Again, it should be noted here that if the ring is flat and extends in the transverse plane, the projection of the ring on the transverse plane then coincides with the ring itself. Furthermore, this results in the surgeon being able to feel when the widest point between the pins has been passed when fitting the securing device on the target vessel since the resistance which has to be overcome is reduced. The surgeon can then, by feel, accurately determine whether the fitting has been carried out correctly.

In order to prevent excessive flattening of the target vessel at the location of the anastomosis, it is advantageous, according to the invention, if the distance between the pins is at most equal to the diameter of the ring.

According to a further embodiment, the distance between the free ends is at least equal to the radius of the ring, in particular said distance is at least equal to 1.5x the radius of the ring. This prevents the target vessel from tearing when the pins are being inserted due to excessive stretching of the section of the wall of the target vessel between the pins.

In order to ensure that the wall of the target vessel between the attachment points does not sag, it is advantageous, according to the invention, if the distance between the attachment sites of the pins is greater than or equal to the distance between the free ends of the pins. It is advantageous if both these distances are equal or approximately equal. This offers the advantage that the pierced holes are virtually stress-free when the securing device has been fitted.

According to a further embodiment of the invention, the pins extend substantially under the transverse plane while the free ends of the pins are situated above the transverse plane. Thus, in the fitted state, the pointed ends can project outwards through the wall of the target vessel. The pointed ends may, if desired, be covered in order to protect the surrounding tissue from the tips, or be treated in another manner.

According to yet a further embodiment of the invention, a tangent axis is defined as extending parallel to the first transverse axis and touching the ring; and a projection length is defined as the distance of the free ends to the tangent axis, which projection length is greater than 0.05 mm. This ensures that the pointed ends of the pins project outside the contour of the ring, which offers a good view of the pointed ends during fitting. It is particularly advantageous for said good view if the projection length is at least 0.5x the radius of the ring, in particular at least 1x the radius of the ring. Even when a distal end portion of the wall of the graft vessel is folded back around the ring, the pins will project beyond the thickening, which results from this folding back, and remain visible. As the thickness of the wall of a graft vessel is usually considerably thinner than 0.5 mm, the projection length can be assumed to be at least 0.5 mm. In order not to affect the flexibility of the target vessel too much, it is in this case furthermore advantageous, according to the invention, if the projection length is at most 3x the radius of the ring, preferably at most 2x the radius of the ring.

According to yet a further embodiment of the invention, the perpendicular projection of a pin on the transverse plane, over a coinciding zone, coincides with the perpendicular projection of the ring on the transverse plane; and the distance of the pin to the ring in said coinciding zone is substantially at least 0.2 mm and at most 1.5 mm, in particular at most 0.7 mm. Said distance may, for example, be approximately 0.5 mm. This ensures, for graft vessels and target vessels of different dimensions, that, in the coinciding zone, both the vessel wall of the graft vessel and the vessel wall of the target vessel together fit between the ring and the pin in a clamping manner.

According to a further embodiment, the ring and pins are made from a single uninterrupted piece of wire, with the pins, from the attachment sites, merging with the ring via a twisted winding in a section of the ring. Thus, it is ensured in a very reliable manner that the pins cannot become detached at the attachment sites of the ring.

According to a further aspect, the invention relates to an assembly comprising:
■ a securing device according to the invention; and
■ a graft vessel;
in which the distal end of the graft vessel is inserted in the ring.
In this case, the pins may be inserted into the section of the wall of the graft vessel which is distal from the ring, but preferably, the pins run through incisions provided in the distal end of the graft vessel.
With this assembly, the graft vessel may be either an artificial vessel, such as an artificial blood vessel, or a (natural) donor vessel, which may be of animal origin or of human origin. Preferably, the graft vessel will be taken from the patient himself or be an artificial vessel. The graft vessel and the securing device can be prepared so as to form said assembly completely outside of the patient's body. This may be carried out in a laboratory at a distance from the patient, even while the patient is not being treated and is, for example, at home. However, this can also be carried out next to the patient while the patient is on the operating table before the operation.

According to a further embodiment of the assembly according to the invention, the section of the wall of the graft vessel which is distal from the ring, at least on the second side of the transverse plane, is folded back until it bears against the section of the wall of the graft vessel which is proximal to the ring. Thus, the prepared assembly can be attached to the target vessel while allowing a good view of the pins.

According to yet a further embodiment of the assembly, it is advantageous in this case if the projection length is at least 1x the thickness of the wall of the graft vessel, preferably at least 2x the thickness of the wall of the graft vessel. Thus, a sufficiently good view of the pointed ends of the pins is ensured during fitting on the target vessel.

According to another further embodiment of the assembly, the assembly furthermore comprises a catheter, in particular a laser catheter, with the graft vessel being pushed onto the distal end of the catheter. This assembly may also be prepared completely outside the patient's body. The catheter which has already been pushed into the graft vessel makes it easier to handle the graft vessel when the pins are inserted into the target vessel.

The present invention will be explained in more detail with reference to embodiments which are diagrammatically illustrated in the drawing, in which:
fig. 1 shows a bottom view (fig. 1A) and side view (fig. 1B) in accordance with arrow Ib of a first embodiment of the securing device according to the invention;
fig. 2 shows a bottom view (fig. 2A) and a side view (fig. 2B) in accordance with arrow IIb of a second embodiment of the securing device according to the invention;
fig. 3 shows a bottom view (fig. 3A) and side view (fig. 3B) in accordance with arrow IIIb of a third embodiment of the securing device according to the invention;
fig. 4 shows a bottom view (fig. 4A) and side view (fig. 4B) in accordance with arrow IVb of a fourth embodiment of the securing device according to the invention;
fig. 5 shows a bottom view (fig. 5A) and a side view (fig. 5B) in accordance with arrow Vb of a fifth embodiment of the securing device according to the invention;
fig. 6 shows a bottom view (fig. 6A) and side view (fig. 6B) in accordance with arrow VIb of a sixth embodiment of the securing device according to the invention;
fig. 7 shows a perspective bottom view of an assembly according to the invention;
fig. 8 shows, in 3 steps (figs. 8A, 8B and 8C, respectively), how a graft vessel can be attached to a target vessel by means of the securing device according to the invention;
fig. 9 shows a longitudinal section (fig. 9A) and end view (fig. 9B) of a further assembly according to the invention; and
fig. 10 shows a top view of a preferred manner in which the securing device is made from a single piece of wire.

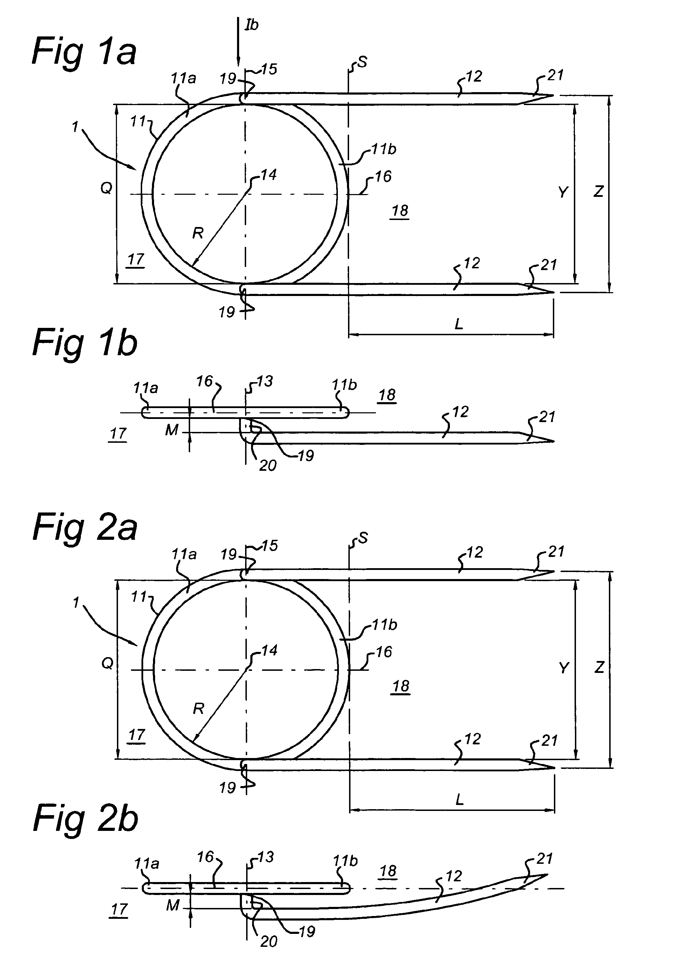

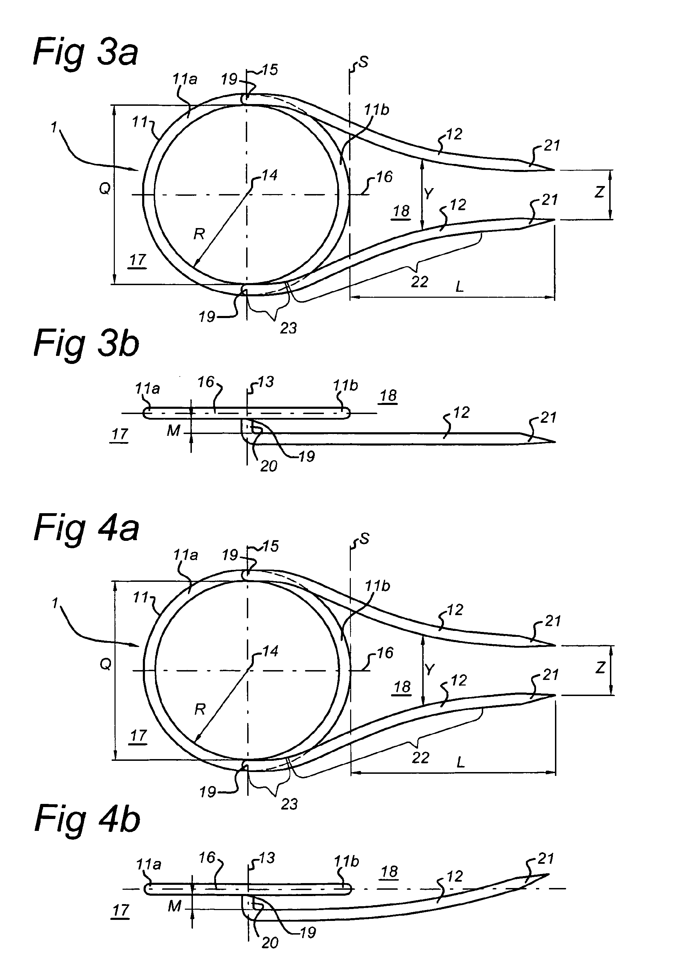

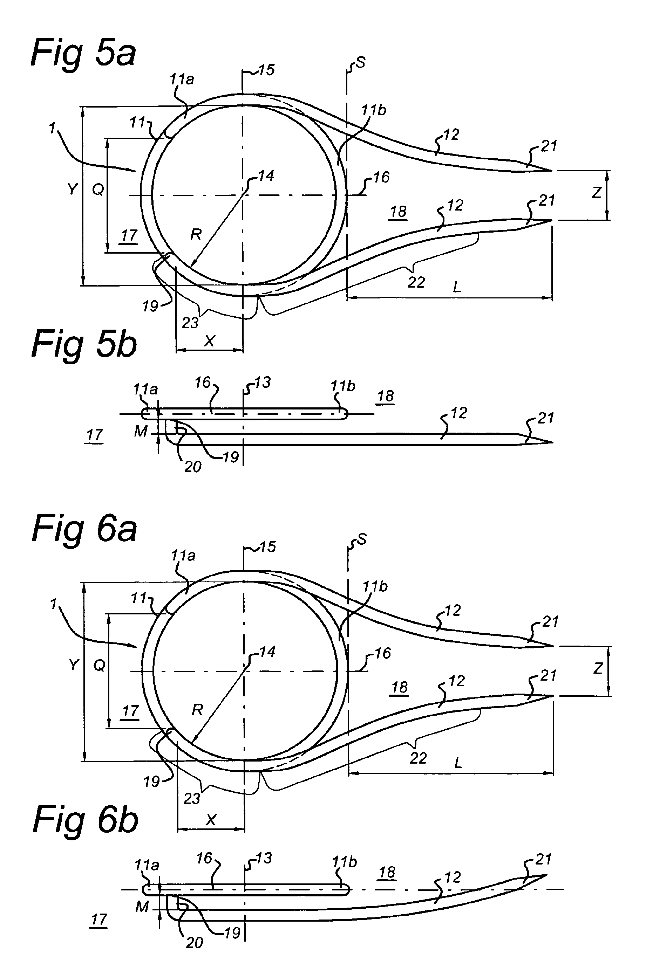

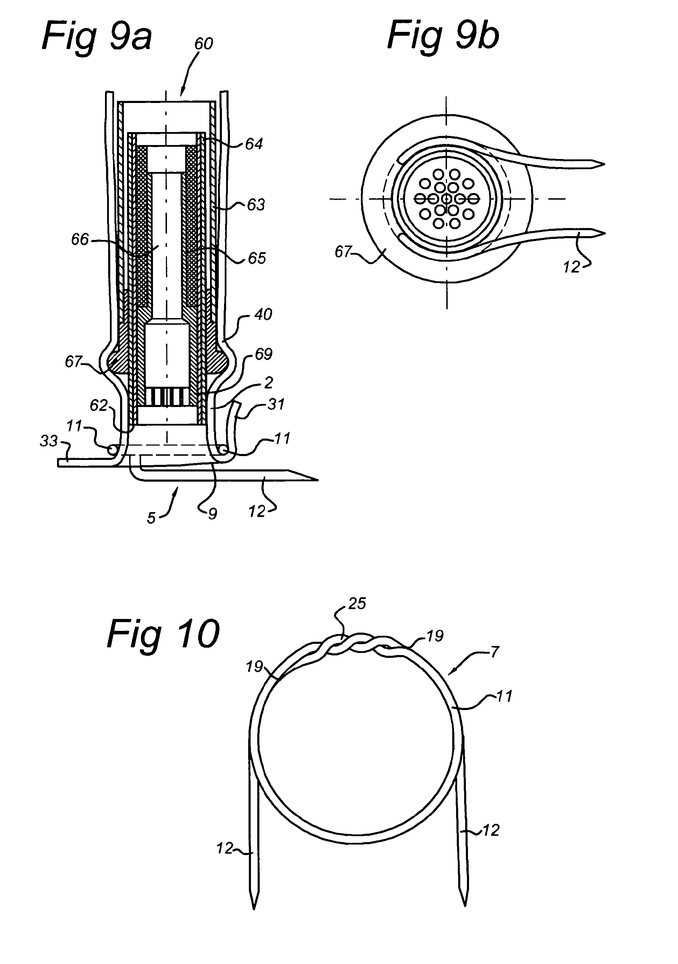

Figs. 1 to 6 show six variants of embodiments of the securing device according to the invention. The variant in fig. 1 is denoted overall by reference numeral 1, the variant in fig. 2 is denoted overall by reference numeral 2, the variant in fig. 3 is denoted overall by reference numeral 3, the variant in fig. 4 is denoted overall by reference numeral 4, the variant in fig. 5 is denoted overall by reference numeral 5, and the variant in fig. 6 is denoted overall by reference numeral 6. For the remainder, identical parts in figs. 1 to 6 are also denoted by the same reference numerals. The variant from fig. 5, preferably in combination with the manufacturing procedure using a single wire from fig. 10, is the embodiment which, in the Applicant's current opinion, is the most preferred embodiment.

Fig. 1 shows a securing device 1 comprising a ring 11 and two pins 12.

The ring 11 has an axial axis 13 which passes through the center 14 of the ring and is at right angles to the plane surrounded by the ring. Furthermore, the ring has a first transverse axis 15 and a second transverse axis 16. The first transverse axis 15, the second transverse axis 16 and the axial axis 15 together define an orthogonal axial system, that is to say they are all at right angles with respect to one another.

The first transverse axis 15 and the second transverse axis 16 together define a transverse plane which, in figs. 1A, 2A, 3A, 4A, 5A and 6A, extends in each case parallel to the plane of the drawing and, in figs. 1B, 2B, 3B, 4B, 5B and 6B, is in each case at right angles to the plane of the drawing. The first transverse axis 15 divides the transverse plane into a first side 17- in each case to the left of the transverse axis 15 in figs. 1-6 - and a second side 18 - in each case to the right of the transverse axis 15 in figs. 1-6. The first transverse axis 15 divides the ring 11 further into a first half 11a (in each case the left half in figs. 1-6) and a second half 11 b (in each case the right half in figs. 1-6). It should be noted that the boundary between the halves determined by the transverse axis 15 is part of the first half 11a.

The pins 12 each have a fixed end 20 and a free end 21, which is pointed. Each pin 12 is attached to the ring by the fixed end 21 at an attachment site 19, in such a manner that the pin 12 extends from the ring 12 in a cantilevered manner as a cantilever pin. As can be seen in fig. 1 (and in figs. 2-6, as well), the free ends of the pins are on the righthand side of the transverse axis 15, or in other words on the second side of the transverse plane which is defined by the first transverse axis 15 and second transverse axis 16. The attachment sites 19 are situated on the other side 17 of the transverse plane near, in this embodiment, the transverse axis 15. The pins 12 are thus attached to the first half 11a of the ring 11. The pins 12 therefore extend from one side - first side 17 - of the transverse plane to the other side - second side 18 - of the transverse plane.

Furthermore, in the figures, R denotes the radius of the ring 11; S denotes a tangent line on the ring which runs parallel to the transverse axis 15; L denotes the projection length of the pins, that is to say the distance of the ends of the pins 12 up to the tangent line S; M denotes the distance of the pin 12 up to the ring 11, viewed in the direction of the axial axis; Q denotes the distance between the attachment sites 19; Y denotes the distance between the pins 12, viewed in the direction of transverse axis 15; Z denotes the distance between the pointed ends of the pins 12, viewed in the direction of transverse axis 15; and X denotes the perpendicular distance of the attachment site 19 to the transverse axis 15.

Fig. 1 shows that the projection length L is greater than the radius R of the ring 11 and is smaller than 3x the radius R of the ring 11. L is shown here as being approximately 2R, but this length may very well be shorter.

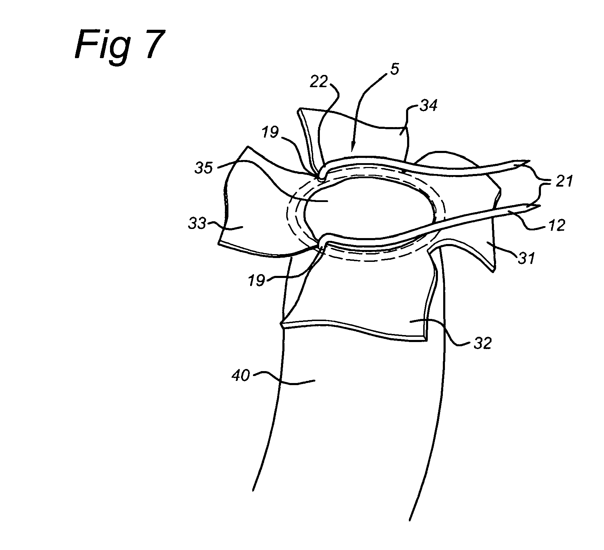

The securing device 2 from fig. 2 differs from the securing device 1 from fig. 1 in that, with the securing device 1, the pins 12 extend substantially parallel to the transverse plane, while with the securing device 2, the pins 12 initially run underneath the transverse plane, are curved upwards towards the transverse plane and project above the transverse plane (the position of which in fig. 2B corresponds to the position of the second transverse axis 16) with their free ends. The advantage thereof is that, after having first been inserted by being pushed through a vessel wall 30 (see fig. 7), the pins (12) stick out of the vessel wall 30 again by their pointed ends 21 (not shown in fig. 7).

The securing device 3 from fig. 3 differs from the securing device 1 from fig. 1 in that the pins 12, viewed from the fixed ends 20 towards the free ends 21, taper towards one another - see the indicated tapering zone 22. The result thereof is that the pointed ends 21 can be inserted into a vessel wall at a relatively close distance to one another - at an intermediate distance Z - and that the pierced holes - i.e. where the pointed ends 21 have penetrated the vessel wall - will be pulled apart when the pointed ends are pushed in further and through the vessel wall, due to the fact that the distance Y between the pins increases. The vessel wall is thus stretched.

The securing device 4 from fig. 4 is substantially a combination of the securing device 3 from fig. 3 and the securing device 2 from fig. 2. The pins 12 both taper towards one another and run upwards so that their pointed ends project above the transverse plane. The securing device 5 from fig. 5 differs from the securing device 3 from fig. 3 in that, with the securing device 5, the attachment sites 19 are situated at a distance X from the first transverse axis 15, and in that the section of the pins 12 which extends along the first side 17 of the transverse plane extends exactly underneath the first half of the ring. As the ring 11 lies in the transverse plane, the perpendicular projection of the ring on said transverse plane coincides with the ring. In the case of the perpendicular projection of the pins 12 on the transverse plane, said projection in the first side 17 of the transverse plane thus coincides with the ring (or the perpendicular projection of the ring on that same transverse plane, respectively). The advantage of positioning the attachment sites 19 at a distance X to the first transverse axis is inter alia that the vessel wall is stretched (in the extending direction of the first transverse axis 15) across a larger section of the ring, and that pulling the securing device out of the vessel wall of the target vessel (in the extending direction of the second transverse axis 16) is rendered more difficult.

The securing device 6 from fig. 6 is essentially a combination of the securing device 5 from fig. 5 and the securing device 4 from fig. 4. In addition to the attachment sites being provided at a distance X, the pins 12 also extend upwards so that they project above the transverse plane by their pointed ends 21.

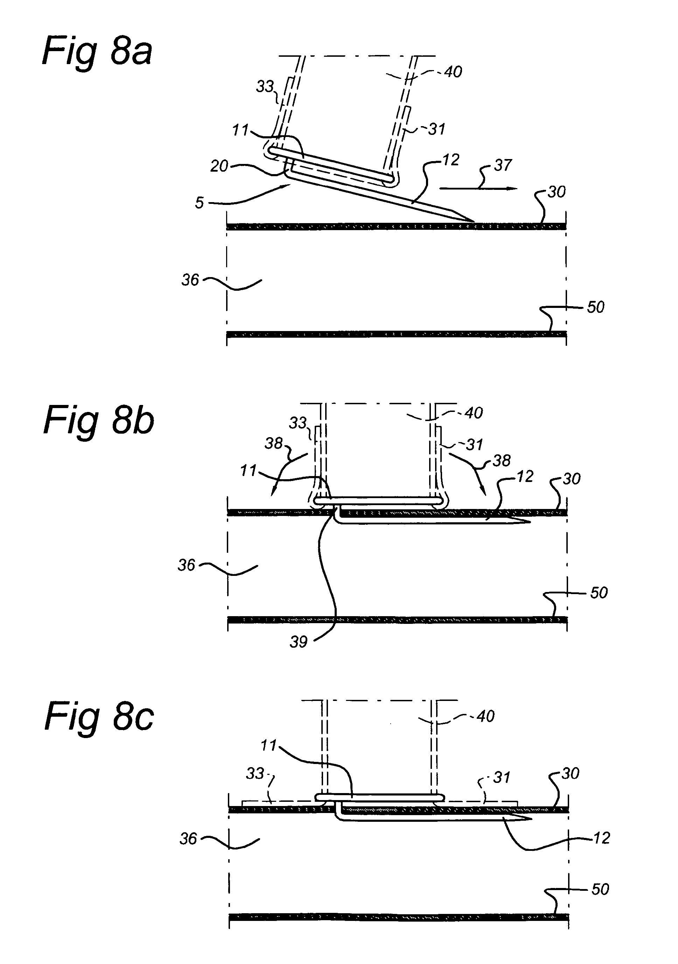

Fig. 7 diagrammatically shows a perspective view of an assembly according to the invention. This assembly comprises a graft vessel 40 and a securing device 5, which, in the Applicant's current opinion, is preferred. Incisions have been made in the distal end of the graft vessel 40 so that four- or more or fewer, such as in particular two - flaps 31, 32, 33, 34 are created. After the distal end of the graft vessel 40 has been inserted in the ring 11, flap 33 is formed by cutting the distal end or, if flap 33 has already been formed beforehand, the distal end of the graft vessel 40 is positioned in such a manner, if this is still necessary, that the flap 33 is situated exactly between the attachment sites 19. The flaps 32, 33 and 34 may, if desired, form a single entity, in which case two further incisions become obsolete. The flaps 31, 32, 33 and 34 are folded back around the ring as a result of which the ring 11 is hidden from view here. In Fig. 7, the flaps are shown as being folded back over approximately 90°. In practice, the flaps, in particular flap 31, may be folded back up to approximately 180° during the attachment on a target vessel 50 discussed in fig. 8 in order to maintain a good view of the pins 12. If the pins 12 are longer than the flap 31, as is illustrated in fig. 7, said folding back up to 180° is still advantageous, although folding back over 90° may already be sufficient in this case. The ring 11 keeps the lumen 35 of the graft vessel 40 at the distal end of the graft vessel 40 open. The pins 12 have been inserted into flaps 34 and 32, respectively, and extend from the fixed ends 22 towards the free ends 21. Incidentally, it should be noted that folding back the distal end of the graft vessel around the ring, according to the invention, is also possible without making any incision (in which case no separate flap is formed). In that case, the pins 12 will then be pushed through the wall of the graft vessel.

Fig. 8, in a number of figs. 8A, 8B and 8C, diagrammatically shows the way in which the assembly according to the invention, as illustrated in for example fig. 7, can be attached to a target vessel 50.

The pins 12 are, see fig. 8A, pushed through the wall 30 of the target vessel 50, in accordance with arrow 37, while fluid, such as blood, flows through the lumen 36. When the pins 12 have reached the lumen 36 of the target vessel 50, the pins are pushed in further until the pierced holes 39 - where the pins 12 enter the target vessel - are situated at the attachment site so that the fixed ends 20 of the pins 12 then extend through the vessel wall. This situation is illustrated in fig. 8b.

Then, the flaps 31, 32, 33 and 34, see arrows 38, are laid flat against the outside of the wall 30 of the target vessel and attached thereto, for example by gluing and/or stitching. This results in the situation as illustrated in fig. 8C.

Fig. 9 shows a further assembly according to the invention. This assembly comprises a graft vessel 40, a securing device 5 and a laser catheter 60 which has been inserted into the graft vessel 40. The combination of graft vessel 40 and securing device 5 has already been discussed with reference to fig. 7. The laser catheter 60 is essentially known per se from the abovementioned EP 750,476, in which the ELANA technique is described. For a detailed description of the ELANA technique and said laser catheter, reference should therefore be made to EP 750,476. Briefly, the laser catheter comprises a bundle of optical fibers 64 through which laser light can be conducted. Said laser light then emerges at the distal end 62 of the fibers 64 and can, if the distal end has been placed against the vessel wall tissue, able to burn a ring of vessel wall tissue away and thus to produce a passageway between a graft vessel and a target vessel after the graft vessel and target vessel have first been connected to one another. Thus, the vessel wall part 37 from fig. 8C can be removed in order to produce the connection. A sleeve 63 is provided around the bundle of fibers, which sleeve 63 is provided with a thickening 67 at the distal end. Inside the bundle of fibers 64, there is a suction tube 65 with a duct 66 which, during use of the laser, is under a vacuum or at least a partial vacuum. Thus, the vessel wall part 37 (fig. 8C) which is burnt away is securely sucked onto the suction nozzle 69 so that the burnt away vessel wall part 37 can be removed at the same time as the catheter 60 is removed. Pulling the burnt-away vessel wall part 37, which is to be burnt away, by the pins 12 has the advantage that said burnt-away vessel wall part can be sucked up more reliably by the suction nozzle 69.

Fig. 10 shows a top view of a securing device 7 according to the invention which is made from a single, uninterrupted wire. An annular loop is formed in the wire and at the location where the further wire parts converge, they are twisted together in a zone of the ring, and the wire ends are bent to form pins of the desired shape. Here, the pins 12 are depicted as running parallel with one another, but preferably, the pins 12 will be shaped in accordance with the embodiment from fig. 5. The ring 11 and pins 12 are thus made from a single uninterrupted piece of wire, with the pins 12, from the attachment sites 19, merging into said ring 11 by means of a twisted winding 25 over part of the ring.

Diameters for the target vessel and graft vessel which are commonly used in practice vary between 1.5 mm and 6 mm, but smaller and larger diameters can also be used. The - internal - diameter of the ring will then vary accordingly, depending on the diameter of the graft vessel to be used. The wall thickness of target vessels is in practice usually in the range of 0.1 to 0.8 mm, and the wall thicknesses of graft vessels are in practice usually in the range from 0.1 to 0.6 mm, but smaller and larger wall thicknesses are also possible for both. Furthermore, the securing device according to the invention can readily be used in neurosurgery, cardiac surgery and general vascular surgery.

## Claims

1. A securing device (1,2,3,4,5,6,7) for an anastomosis connection, comprising a ring (11) provided with two cantilever pins (12);
in which the ring (11) has an axial axis (13) which is defined as an axis at right angles to the annular plane surrounded by the ring and through the center (14) of the ring (10);
in which the ring (11) has a first transverse axis (15) and a second transverse axis (16), which are at right angles to one another and are both also at right angles to the axial axis (13), and which together establish a transverse plane;
in which the first transverse axis divides the ring (11) into a first half (11a) and a second half (11b);
in which the transverse plane has a first side (17) along which the first half (11a) of the ring (11) extends and a second side (18) along which the second half (11b) of the ring (11) extends;
in which each cantilever pin (12), at in each case one attachment site (19), is connected to the ring by a fixed end (20) in order, in a cantilevered manner, to extend from the ring (11) to the other, free end (21) of the pin, which free end (21) is pointed;
in which, viewed from the ring (11), the pins (12) extend in the same direction;
in which, viewed in the transverse plane, the free ends (21) of the pins (11) are situated on the second side (18) of the transverse plane;
in which the attachment sites (19) are situated on the first half (11a) of the ring (11).

2. The securing device (3, 4, 5, 6, 7) as claimed in claim 1, in which the sections of the pins (12) extending along the second side (18) of the transverse plane have a tapering zone (22) in which said pins (12), viewed in the direction of the free ends (21) thereof, taper towards one another.

3. The securing device (3, 4, 5, 6, 7) as claimed in claim 2, in which, in said tapering zone (22), the perpendicular projection of said pins (12) on said transverse plane at least partially coincides with the perpendicular projection of the ring (11) on said transverse plane.

4. The securing device (5, 6, 7) as claimed in one of the preceding claims, in which the attachment site (19) of both pins (12) is situated at a distance (X) of the first transverse axis (15).

5. The securing device (5, 6, 7) as claimed in claim 4, in which said distance (X) to the first transverse axis (15) is at least 10% of the radius (R) of the ring (11), in particular at least 25% of the radius (R) of the ring, more particularly at least 50% of the radius (R) of the ring (11), even more particularly at least 70% of the radius (R) of the ring (11).

6. The securing device (5,6,7) as claimed in claim 4 or 5, in which, in the first side (17) of the transverse plane, the perpendicular projection of the pins (12) on said transverse plane coincides with the perpendicular projection of the ring (11) on said transverse plane.

7. The securing device (1, 2, 3, 4, 5, 6, 7) as claimed in one of the preceding claims, in which the distance (Y) between the pins is at most equal to the diameter of the ring.

8. The securing device (1, 2, 3, 4, 5, 6, 7) as claimed in one of the preceding claims, in which the distance (Z) between the free ends (21) is at least equal to the radius (R) of the ring, and in particular is at least equal to 1.5x the radius (R) of the ring.

9. The securing device (3, 4, 5, 6, 7) as claimed in one of the preceding claims, in which the distance (Q) between the attachment sites (19) of the pins (12) is at least equal to the distance (Z) between the free ends (21) of the pins (12).

10. The securing device (2, 4, 6, 7) as claimed in one of the preceding claims, in which the pins (12) extend substantially under the transverse plane while the free ends (21) of the pins are situated above the transverse plane.

11. The securing device (1, 2, 3, 4, 5, 6, 7) as claimed in one of the preceding claims, in which a tangent axis (S) is defined as extending parallel to the first transverse axis (15) and touching the ring; and
in which a projection length (L) is defined as the distance of the tip of the free ends (21) to the tangent axis, which projection length (L) is greater than 0.00 mm.

12. The securing device (1, 2, 3, 4, 5, 6, 7) as claimed in claim 11, in which the projection length (L) is at least 0.5x the radius (R) of the ring (11), in particular at least 1x the radius (R) of the ring (11).

13. The securing device (1,2,3,4,5,6,7) as claimed in one of claims 11-12, in which the projection length (L) is at least 0.5 mm.

14. The securing device (1, 2, 3, 4, 5, 6, 7) as claimed in one of claims 11 - 13 , in which the projection length (L) is at most 3x the radius (R) of the ring (11), preferably at most 2x the radius (R) of the ring (11).

15. The securing device (1,2,3,4,5,6,7) as claimed in one of the preceding claims, in which the perpendicular projection of a pin (12) on the transverse plane, over a coinciding zone, coincides with the perpendicular projection of the ring on the transverse plane; and in which the distance (M) of the pin to the ring in said coinciding zone (23) is substantially at least 0.2 mm and at most 1.5 mm, in particular at most 0.7 mm.

16. The securing device (7) as claimed in one of the preceding claims, in which the ring (11) and pins (12) are made from a single uninterrupted piece of wire, and with the pins (12), from the attachment sites (19), merging with the ring (11) via a twisted winding (25) in a section of the ring.

17. An assembly comprising:
■ a securing device as claimed in one of the preceding claims; and
■ a graft vessel;
in which the distal end of the graft vessel is inserted in the ring.

18. The assembly as claimed in claim 17, in which the section of the wall of the graft vessel which is distal from the ring, at least on the second side of the transverse plane, is folded back until it bears against the section of the wall of the graft vessel which is proximal to the ring.

19. The assembly as claimed in claim 17 or 18, in combination with one of claims 12-14, in which the projection length is at least 1x the thickness of the wall of the graft vessel, preferably at least 2x the thickness of the wall of the graft vessel.

20. The assembly as claimed in one of claims 17-19, furthermore comprising a catheter, in particular a laser catheter, with the graft vessel being pushed onto the distal end of the catheter.

## Patentansprüche

1. Befestigungsvorrichtung (1, 2, 3, 4, 5, 6, 7) für eine Anastomose-Verbindung, die einen Ring (11) umfasst, der mit zwei Auslegerstiften (12) versehen ist;
wobei der Ring (11) eine axiale Achse (13) hat, die als eine Achse im rechten Winkel zu der ringförmigen Ebene, die von dem Ring umgeben wird, sowie durch die Mitte (14) des Rings (11) hindurch ausgebildet ist;
wobei der Ring (11) eine erste Querachse (15) und eine zweite Querachse (16) hat, die im rechten Winkel zueinander und beide auch im rechten Winkel zu der axialen Achse (13) sind, und die zusammen eine Querebene bilden;
wobei die erste Querachse den Ring (11) in eine erste Hälfte (11a) und eine zweite Hälfte (11b) teilt;
wobei die Querebene eine erste Seite (17), an der die erste Hälfte (11a) des Rings (11) entlang verläuft und eine zweite Seite (18) hat, an der die zweite Hälfte (11b) des Rings (11) entlang verläuft;
wobei jeder Auslegerstift (12) an in jedem Fall einer Anbringungsstelle (19) über ein stationäres Ende (20) mit dem Ring verbunden ist, so dass er sich auslegerartig von dem Ring (11) zu dem anderen, freien Ende (21) des Stiftes erstreckt, wobei das freie Ende (21) spitz ist;
wobei sich die Stifte (12), von dem Ring (11) aus gesehen, in der gleichen Richtung erstrecken;
wobei sich die freien Enden (21) der Stifte (12), in der Querebene gesehen, an der zweiten Seiten (18) der Querebene befinden;
und wobei sich die Anbringungsstellen (19) an der ersten Hälfte (11a) des Rings (11) befinden.

2. Befestigungsvorrichtung (3, 4, 5, 6, 7) nach Anspruch 1, wobei die Abschnitte der Stifte (12), die sich an der zweiten Seite (18) der Querebene entlang erstrecken, eine sich verjüngende Zone (22) haben, in der sich die Stifte (12), in der Richtung der freien Enden (21) derselben gesehen, aufeinander zu verjüngen.

3. Befestigungsvorrichtung (3, 4, 5, 6, 7) nach Anspruch 2, wobei sich die senkrechte Projektion der Stifte (12) auf der Querebene in der Verjüngungszone (22) wenigstens teilweise mit der senkrechten Projektion des Rings (11) auf der Querebene deckt.

4. Befestigungsvorrichtung (5, 6, 7) nach einem der vorangehenden Ansprüche, wobei sich die Anbringungsstelle (19) beider Stifte (12) in einem Abstand (X) zu der ersten Querachse (15) befindet.

5. Befestigungsvorrichtung (5, 6, 7) nach Anspruch 4, wobei der Abstand (X) zu der ersten Querachse (15) wenigstens 10 % des Radius (R) des Rings (11), insbesondere wenigstens 25 % des Radius (R) des Rings, im Besonderen wenigstens 50 % des Radius (R) des Rings (11) und namentlich wenigstens 70 % des Radius (R) des Rings (11) beträgt.

6. Befestigungsvorrichtung (5, 6, 7) nach Anspruch 4 oder 5, wobei sich die senkrechte Projektion der Stifte (12) auf der Querebene an der ersten Seite (17) der Querebene mit der senkrechten Projektion des Rings (11) auf der Querebene deckt.

7. Befestigungsvorrichtung (1, 2, 3, 4, 5, 6, 7) nach einem der vorangehenden Ansprüche, wobei der Abstand (Y) zwischen den Stiften maximal dem Durchmesser des Rings entspricht.

8. Befestigungsvorrichtung (1, 2, 3, 4, 5, 6, 7) nach einem der vorangehenden Ansprüche, wobei der Abstand (Z) zwischen den freien Enden (21) wenigstens dem Radius (R) des Rings und insbesondere wenigstens dem 1,5-fachen des Radius (R) des Rings entspricht.

9. Befestigungsvorrichtung (3, 4, 5, 6, 7) nach einem der vorangehenden Ansprüche, wobei der Abstand (Q) zwischen den Anbringungsstellen (19) der Stifte wenigstens dem Abstand (Z) zwischen den freien Enden (21) der Stifte (12) entspricht.

10. Befestigungsvorrichtung (2, 4, 6, 7) nach einem der vorangehenden Ansprüche, wobei sich die Stifte (12) im Wesentlichen unter der Querebene erstrecken, während sich die freien Enden (21) der Stifte oberhalb der Querebene befinden.

11. Befestigungsvorrichtung (1, 2, 3, 4, 5, 6, 7) nach einem der vorangehenden Ansprüche,
wobei eine Tangentenachse (S) so ausgebildet ist, dass sie parallel zu der ersten Querachse (15) verläuft und den Ring berührt; und
wobei eine Vorstehlänge (L) als der Abstand der Spitze der freien Enden (21) zu der Tangentenachse definiert ist und die Vorstehlänge (L) größer ist als 0,00 mm.

12. Befestigungsvorrichtung (1, 2, 3, 4, 5, 6, 7) nach Anspruch 11, wobei die Vorstehlänge (L) wenigstens das 0,5-fache des Radius (R) des Rings (11), insbesondere wenigstens das 1-fache des Radius (R) des Rings (11), beträgt.

13. Befestigungsvorrichtung (1, 2, 3, 4, 5, 6, 7) nach einem der Ansprüche 11-12, wobei die Vorstehlänge (L) wenigstens 0,5 mm beträgt.

14. Befestigungsvorrichtung (1, 2, 3, 4, 5, 6, 7) nach einem der Ansprüche 11-13, wobei die Vorstehlänge (L) maximal das 3-fache des Radius (R) des Rings (11), vorzugsweise maximal das 2-fache des Radius (R) des Rings (11), beträgt.

15. Befestigungsvorrichtung (1, 2, 3, 4, 5, 6, 7) nach einem der vorangehenden Ansprüche, wobei sich die senkrechte Projektion eines Stiftes (12) auf der Querebene über eine Deckungszone mit der senkrechten Projektion des Rings auf der Querebene deckt, und der Abstand (M) des Stiftes zu dem Ring in der Deckungszone (23) im Wesentlichen wenigstens 0,2 mm und maximal 1,5 mm, insbesondere maximal 0,7 mm, beträgt.

16. Befestigungsvorrichtung (7) nach einem der vorangehenden Ansprüche, wobei der Ring (11) und die Stifte (12) aus einem einzelnen ununterbrochenen Stück Draht bestehen und die Stifte (12) von den Anbringungsstellen (13) über eine verdrehte Windung (25) in einem Abschnitt des Rings in den Ring (11) übergehen.

17. Anordnung, die umfasst:
eine Befestigungsvorrichtung nach einem der vorangehenden Ansprüche; und
ein implantiertes Gefäß;
wobei das distale Ende des implantierten Gefäßes in den Ring eingeführt wird.

18. Anordnung nach Anspruch 17, wobei der Abschnitt der Wand des implantierten Gefäßes, der distal zu dem Ring liegt, wenigstens an der zweiten Seite der Querebene zurückgeklappt wird, bis er an dem Abschnitt der Wand des implantierten Gefäßes anliegt, die proximal zu dem Ring liegt.

19. Anordnung nach Anspruch 17 oder 18 in Kombination mit einem der Ansprüche 12-14, wobei die Vorstehlänge wenigstens das 1-fache der Dicke der Wand des implantierten Gefäßes, vorzugsweise wenigstens das 2-fache der Dicke der Wand des implantierten Gefäßes, beträgt.

20. Anordnung nach einem der Ansprüche 17-19, die des Weiteren einen Katheter, insbesondere einen Laser-Katheter, umfasst, wobei das implantierte Gefäß auf das distale Ende des Katheters gedrückt wird.

## Revendications

1. Dispositif de fixation (1, 2, 3, 4, 5, 6, 7) pour un raccordement d'anastomose, comprenant un anneau (11) prévu avec deux broches en porte-à-faux (12) ;
dans lequel l'anneau (11) a un axe axial (13) qui est défini comme étant un axe en angle droit par rapport au plan annulaire entouré par l'anneau et passant par le centre (14) de l'anneau (10) ;
dans lequel l'anneau (11) a un premier axe transversal (15) et un deuxième axe transversal (16) qui sont en angle droit l'un par rapport à l'autre et sont tous deux également en angle droit par rapport à l'axe axial (13), et qui établissent ensemble un plan transversal ;
dans lequel le premier axe transversal divise l'anneau (11) en une première moitié (11a) et en une deuxième moitié (11b) ;
dans lequel le plan transversal a un premier côté (17) le long duquel la première moitié (11a) de l'anneau (11) s'étend et un deuxième côté (18) le long duquel la deuxième moitié (11b) de l'anneau (11) s'étend ;
dans lequel chaque broche en porte-à-faux (12), dans chaque cas, au niveau d'un site de fixation (19), est raccordée à l'anneau par une extrémité fixe (20), afin de s'étendre, en porte-à-faux, à partir de l'anneau (11) jusqu'à l'autre extrémité libre (21) de la broche, laquelle extrémité libre (21) est pointue ;
dans lequel, observées à partir de l'anneau (11), les broches (12) s'étendent dans la même direction ;
dans lequel, observées dans le plan transversal, les extrémités libres (21) des broches (11) sont situées sur le deuxième côté (18) du plan transversal ;
dans lequel les sites de fixation (19) sont situés sur la première moitié (11a) de l'anneau (11).

2. Dispositif de fixation (3, 4, 5, 6, 7) selon la revendication 1, dans lequel les sections des broches (12) s'étendant le long du deuxième côté (18) du plan transversal ont une zone progressivement rétrécie (22) dans laquelle lesdites broches (12), observées dans la direction de leurs extrémités libres (21), se rétrécissent progressivement l'une vers l'autre.

3. Dispositif de fixation (3, 4, 5, 6, 7) selon la revendication 2, dans lequel, dans ladite zone progressivement rétrécie (22), la saillie perpendiculaire desdites broches (12) sur ledit plan transversal coïncident au moins partiellement avec la saillie perpendiculaire de l'anneau (11) sur ledit plan transversal.

4. Dispositif de fixation (5, 6, 7) selon l'une quelconque des revendications précédentes, dans lequel le site de fixation (19) des deux broches (12) est situé à une distance (X) du premier axe transversal (15).

5. Dispositif de fixation (5, 6, 7) selon la revendication 4, dans lequel ladite distance (X) jusqu'au premier axe transversal (15) représente au moins 10% du rayon (R) de l'anneau (11), en particulier au moins 25% du rayon (R) de l'anneau, plus particulièrement au moins 50% du rayon (R) de l'anneau (11), encore plus particulièrement au moins 70% du rayon (R) de l'anneau (11).

6. Dispositif de fixation (5, 6, 7) selon la revendication 4 ou 5, dans lequel, dans le premier côté (17) du plan transversal, la saillie perpendiculaire des broches (12) sur ledit plan transversal coïncide avec la saillie perpendiculaire de l'anneau (11) sur ledit plan transversal.

7. Dispositif de fixation (1, 2, 3, 4, 5, 6, 7) selon l'une quelconque des revendications précédentes, dans lequel la distance (Y) entre les broches est au maximum égale au diamètre de l'anneau.

8. Dispositif de fixation (1, 2, 3, 4, 5, 6, 7) selon l'une quelconque des revendications précédentes, dans lequel la distance (Z) entre les extrémités libres (21) est au moins égale au rayon (R) de l'anneau, et en particulier est au moins égale à 1,5x le rayon (R) de l'anneau.

9. Dispositif de fixation (3, 4, 5, 6, 7) selon l'une quelconque des revendications précédentes, dans lequel la distance (Q) entre les sites de fixation (19) des broches (12) est au moins égale à la distance (Z) entre les extrémités libres (21) des broches (12).

10. Dispositif de fixation (2, 4, 6, 7) selon l'une quelconque des revendications précédentes, dans lequel les broches (12) s'étendent sensiblement sous le plan transversal alors que les extrémités libres (21) des broches sont situées au-dessus du plan transversal.

11. Dispositif de fixation (1, 2, 3, 4, 5, 6, 7) selon l'une quelconque des revendications précédentes, dans lequel un axe tangent (S) est défini comme s'étendant parallèlement au premier axe transversal (15) et venant en contact avec l'anneau ; et
dans lequel une longueur de saillie (L) est définie comme étant la distance de la pointe des extrémités libres (21) jusqu'à l'axe tangent, laquelle longueur de saillie (L) est supérieure à 0,00 mm.

12. Dispositif de fixation (1, 2, 3, 4, 5, 6, 7) selon la revendication 11, dans lequel la longueur de saillie (L) représente au moins 0,5x le rayon (R) de l'anneau (11), en particulier au moins 1x le rayon (R) de l'anneau (11).

13. Dispositif de fixation (1, 2, 3, 4, 5, 6, 7) selon l'une quelconque des revendications 11 à 12, dans lequel la longueur de saillie (L) est d'au moins 0,5 mm.

14. Dispositif de fixation (1, 2, 3, 4, 5, 6, 7) selon l'une quelconque des revendications 11 à 13, dans lequel la longueur de saillie (L) représente au maximum 3x le rayon (R) de l'anneau (11), de préférence au maximum 2x le rayon (R) de l'anneau (11).

15. Dispositif de fixation (1, 2, 3, 4, 5, 6, 7) selon l'une quelconque des revendications précédentes, dans lequel la saillie perpendiculaire d'une broche (12) sur le plan transversal, sur une zone de coïncidence, coïncide avec la saillie perpendiculaire de l'anneau sur le plan transversal ; et dans lequel la distance (M) de la broche jusqu'à l'anneau dans ladite zone de coïncidence (23) est sensiblement d'au moins 0,2 mm et au maximum de 1,5 mm, en particulier au maximum de 0,7 mm.

16. Dispositif de fixation (7) selon l'une quelconque des revendications précédentes, dans lequel l'anneau (11) et les broches (12) sont réalisés à partir d'une seule pièce de fil d'un seul tenant, et dont les broches (12), à partir des sites de fixation (19), se rejoignent avec l'anneau (11) via un enroulement torsadé (25) dans une section de l'anneau.

17. Ensemble comprenant :
un dispositif de fixation selon l'une quelconque des revendications précédentes ; et
un greffon Veineux ;
dans lequel une extrémité distale du greffon veineux est insérée dans l'anneau.

18. Ensemble selon la revendication 17, dans lequel la section de la paroi du greffon veineux qui est distale par rapport à l'anneau, au moins sur le deuxième côté du plan transversal, est repliée jusqu'à ce qu'elle s'appuie contre la section de la paroi du greffon veineux qui est proximale par rapport à l'anneau.

19. Ensemble selon la revendication 17 ou 18, en combinaison avec l'une quelconque des revendications 12 à 14, dans lequel la longueur de saillie représente au moins 1x l'épaisseur de la paroi du greffon veineux, de préférence au moins 2x l'épaisseur de la paroi du greffon veineux.

20. Ensemble selon l'une quelconque des revendications 17 à 19, comprenant en outre un cathéter, en particulier un cathéter laser, avec le greffon veineux qui est poussé sous l'extrémité distale du cathéter.
